# EUROPÄISCHE PATENTSCHRIFT

(11) **EP 0 869 113 B1**
(45) Veröffentlichungstag und Bekanntmachung des Hinweises auf die Patenterteilung: **04.06.2003**
(21) Anmeldenummer: 98105636.9
(22) Anmeldetag: 27.03.1998
(51) Int. Cl.: C07C 209/48, C07C 211/12, C07C 213/02, C07C 215/08

(54) **Verfahren zur Herstellung tertiärer Amine aus Nitrilen und sekundären Aminen**
Process for the preparation of tertiary amines fromnitriles and secondary amines
Procédé pour la préparation d'amines tertiaires à partir de nitriles et amines secondaires

(30) Priorität: 01.04.1997 DE 19713383
(43) Veröffentlichungstag der Anmeldung: 07.10.1998
(73) Patentinhaber: BASF AKTIENGESELLSCHAFT, 67056 Ludwigshafen (DE)
(72) Erfinder: Fuchs, Eberhard, Dr., 67227 Frankenthal (DE); Breitscheidel, Boris, Dr., 67117 Limburgerhof (DE); Becker, Rainer, Dr., 67098 Bad Dürkheim (DE); Neuhauser, Horst, Dr., 67373 Dudenhofen (DE)
(74) Vertreter: Isenbruck, Günter, Dr.

(56) Entgegenhaltungen:
- DE-A- 4 239 782
- DE-A- 4 407 466
- DE-A- 19 518 038

## Beschreibung

Die Erfindung betrifft ein Verfahren zur Herstellung von tertiären Aminen aus Nitrilen und sekundären Aminen an einem Palladium-Katalysator.

Verfahren zur Herstellung von tertiären Aminen aus Nitrilen und sekundären Aminen an bestimmten Palladium-Katalysatoren sind bekannt.

In der DE-A-4 239 782 ist ein Verfahren zur Herstellung von Diaminen durch Umsetzung von Dinitrilen mit sekundären Aminen beschrieben. Dabei wird Adipodinitril mit Dimethylamin und Wasserstoff in Gegenwart eines Palladium-Katalysators zu Tetramethylhexamethylendiamin umgesetzt. Verwendete Katalysatoren sind 4% Pd auf Al₂O₃, 0,5% Pd auf Al₂O₃ mit 20% CaO, 1% Pd auf Al₂O₃ mit 20% MgO und 0,5% Pd, 5% Pr auf Al₂O₃.

In der DE-A-4 407 466 ist ein Verfahren zur Herstellung von peralkylierten Aminen beschrieben. Nitrile werden mit sekundären Aminen und Wasserstoff in Gegenwart von Palladium-Katalysatoren zu den peralkylierten Aminen umgesetzt. Dabei werden 3-Dimethylaminopropionitril und Dimethylamin zu Tetramethylpropylendiamin, 3-Hydroxypropionitril und Dimethylamin zu 3-Dimethylaminopropanol und Piperazin mit Acetonitril zu N-Ethylpiperazin umgesetzt. Als Katalysatoren werden 0,5 Gew.-% Pd auf Al₂O₃ mit 20 Gew.-% CaO, 0,5 Gew.-% Pd und 5 Gew.-% Pr auf Al₂O₃ oder 0,5 Gew.-% Pd auf Al₂O₃ verwendet.

Bei den bekannten Katalysatoren sind für manche Anwendungen die Selektivität, der Umsatz bzw. die Standzeit verbesserungswürdig.

DE-A 195 18 038 betrifft ein Verfahren zur Herstellung von N-Methyl-2-(3,4-dimethoxyphenyl)-ethylamin. Zur Herstellung setzt man 3,4-dimethoxyphenylmethylnitril mit Methylamin und Wasserstoff in Gegenwart eines Katalysators um. Der Katalysator enthält Kupferchromit, Kupfer, Silber, Gold, Eisen, Cobalt, Nickel, Ruthenium, Rhodium, Palladium, Osmium, Iridium, Platin oder deren Gemische. Bevorzugt sind Kupferchromit, Kupfer, Palladium, Platin oder deren Gemische, besonders bevorzugt Palladium, Platin oder deren Gemische. Insbesondere bevorzugt ist Platin in einem Gesamtgehalt in der Regel von 0,05 bis 50 Gew.-%, bevorzugt 0,1 bis 20 Gew.-%, besonders bevorzugt 0,5 bis 5 Gew.-%. Gemäß der Beispiele werden Palladium oder Platin allein auf einem oxidischen Träger eingesetzt, nur Katalysator D enthält 0,4 Gew.-% Pd und 0,4 Gew.-% Pt auf SiO₂/Al₂O₃.

In Cerveny, Studies in surface science and catalysis, Bd. 27 (1986), Seiten 105 bis 144 ist beschrieben, daß bei der Herstellung tertiärer Amine aus Nitrilen in Gegenwart eines Platinkatalysators der Umsatz in der Regel aufgrund der starken Adsorption der tertiären Amine an der Katalysatoroberfläche unvollständig ist.

Aufgabe der vorliegenden Erfindung ist die Bereitstellung eines Verfahrens zur Herstellung von tertiären Aminen durch Umsetzung von Nitrilen mit sekundären Aminen und Wasserstoff in Gegenwart eines Pd enthaltenen Katalysators, wobei gegenüber bekannten Verfahren die Standzeit des Katalysators verbessert ist.

Gelöst wird die Aufgabe durch ein Verfahren zur Herstellung von Aminen der allgemeinen Formel (I)

X(-CH₂-NR¹R²)ₙ (I)

in der
- R¹, R²: unabhängig C₁₋₂₀₀-Alkyl, C₃₋₈-Cycloalkyl, C₄₋₂₀-Alkylcycloakyl,C₄₋ ₂₀-Cycloalkylalkyl, C₂₋₂₀-Alkoxyalkyl, Aryl, C₇₋₂₀-Alkylaryl, C_{7-20⁻} Aralkyl, C₂₋₈-Hydroxyalkyl, C₂₋₈-Mercaptoalkyl, C₈₋₂₀-Aryloxyalkyl oder gemeinsam eine gegebenenfalls ein- bis dreifach durch C_{1-4⁻} Alkyl substituierte gesättigte oder ungesättigte gegebenenfalls durch Sauerstoff unterbrochene C₂₋₆-Alkylenkette bedeuten
- X: ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy, C₂₋₈-Alkoxycarbonyl substituiertes C₁₋₂₀-Alkyl, C₂₋₂₀-Alkenyl oder C₃₋₈-Cycloalkyl mit n freien Valenzen ist und
- n: eine ganze Zahl von 1 bis 4 ist,
durch Umsetzung von Nitrilen der allgemeinen Formel (II)

(R¹R²N-CH₂-)ₙ₋ₘX(-CN)ₘ (II)

in der R¹, R² und X die oben angegebenen Bedeutungen haben und m eine ganze Zahl von 1 bis n ist,
mit sekundären Aminen der allgemeinen Formel (III)

HNR¹R² (III)

in der R¹ und R² die oben angegebenen Bedeutungen haben,
und Wasserstoff bei Temperaturen von 50 bis 250°C und Drücken von 5 bis 350 bar in Gegenwart eines Pd enthaltenden Katalysators, wobei der Katalysator, bezogen auf das Gesamtgewicht des Katalysators 0,5 bis 1 Gew.-% Pd und 0,05 bis 0,2 Gew.-% Platin oder 1 bis 5 Gew.-% auf einem Träger enthält.

Erfindungsgemäß wurde gefunden, daß die Verwendung eines wie vorstehend definierten Katalysators zur Umsetzung von sekundären Aminen mit Nitrilen und Wasserstoff zu tertiären Aminen zu einer längeren Standzeit bzw. Langzeitstabilität des Katalysators führt.

Entgegen dem eingangs zitierten Artikel von Cerveny, in dem die Nachteile der Verwendung von Platinkatalysatoren zur Umsetzung von Nitrilen zu tertiären Aminen aufgeführt sind, wurde gefunden, daß durch die Kombination von Palladium und Platin oder Lanthan verbesserte Katalysatoren für das obige Verfahren erhalten werden.

Die erfindungsgemäß eingesetzten Katalysatoren enthalten, bezogen auf das Gesamtgewicht des Katalysators 0,5 bis 1 Gew.-% Palladium und 0,05 bis 0,2 Gew.-% Platin oder 0,5 bis 1 Gew.-% Palladium und 1 bis 5 Gew.-% Lanthan.

Besonders bevorzugt sind ein Katalysator, der etwa 0,9 Gew.-% Pd und etwa 0,1 Gew.-% Pt, bezogen auf das Gesamtgewicht des Katalysators, auf ZrO₂ als Träger enthält, und ein Katalysator, der etwa 0,9 Gew.-% Pd unbd etwa 3 Gew.-% Lanthan, bezogen auf das Gesamtgewicht des Katalysators, auf Al₂O₃ oder SiO₂ als Träger enthält.

Als Träger können alle bekannten geeigneten Träger verwendet werden. Beispielsweise ist der Träger ausgewählt aus Aktivkohle, Siliciumcarbid und Metalloxiden. Dabei werden als Metalloxide vorzugsweise Aluminiumoxid, Siliciumdioxid, Titandioxid, Zirkoniumdioxid, Zinkoxid, Magnesiumoxid oder deren Gemische verwendet, die gegebenfalls mit Alkali- und/oder Erdalkalimetalloxiden dotiert sind. Besonders bevorzugt werden γ-Aluminiumoxid, Siliciumdioxid, Zirkoniumdioxid oder Titanoxid oder Gemische davon eingesetzt.

Die Träger können in beliebiger Form eingesetzt werden, beispielsweise als Extrudate (in Form von Strängen), Pellets oder Tabletten. Die Katalysatoren können nach allgemein bekannten Verfahren hergestellt werden, beispielsweise durch Tränken des Trägers mit Lösungen von Verbindungen der eingesetzten Metalle. Palladium kann beispielsweise durch Tränken des Trägers mit Lösungen von PdCl₂ oder Pd(NO₃)₂ aufgetragen werden.

Die Träger können beispielsweise mit Metall-Vorläufern beschichtet werden. Als Metallvorläufer eignen sich Metallsalze, wie Nitrate, Nitrosylnitrate, Halogenide, Carbonate, Carboxylate, Acetylacetonate, Chlorokomplexe, Nitritokomplexe und Aminkomplexe. Bevorzugt sind Nitrate, Chloride, Chlorokomplexe und Aminkomplexe. Das Aufbringen erfolgt vorzugsweise durch Tränken. Die Metallvorläufer der Metalle können gleichzeitig oder nacheinander aufgetränkt werden. Die Reihenfolge, in der die Aktivkomponenten aufgetränkt werden, ist beliebig wählbar.

Weitere Verfahren zur Herstellung der erfindungsgemäß verwendeten Katalysatoren sind dem Fachmann bekannt und schließen das Bedampfen, Besputtern und gemeinsame Fällen ein.

Die Oberfläche, das Porenvolumen und die Porengrößenverteilung des Katalysators sind in weiten Bereichen unkritisch.

Das erfindungsgemäße Verfahren wird bei Temperaturen von 50 bis 200°C, vorzugsweise 90 bis 170°C, besonders bevorzugt 120 bis 160°C und Drükken von 5 bis 300 bar, vorzugsweise 50 bis 250 bar, besonders bevorzugt 70 bis 210 bar diskontinuierlich oder bevorzugt kontinuierlich in Druckapparaturen wie Autoklaven oder bevorzugt in einem Rohrreaktor durchgeführt. Der Druck ist dabei vorzugsweise der Wasserstoffdruck im Reaktor. Bei der Verwendung eines Rohrreaktors kann der verwendete Katalysator auch als Festbettkatalysator vorliegen.

Der Reaktor wird mit dem Nitril der allgemeinen Formel (II) und dem sekundären Amin der allgemeinen Formel (III) vorzugsweise in einem Molverhältnis, bezogen auf eine Nitrilgruppe, von 1:1 bis 30:1, vorzugsweise 1:1 bis 15:1, besonders bevorzugt 1,1:1 bis 5:1 beschickt. Es können jedoch auch größere Aminüberschüsse oder aber auch Aminunterschüsse eingestellt werden.

Das erfindungsgemäße Verfahren läßt sich lösungsmittelfrei oder in Lösungsmitteln, wie Wasser, Methanol, Ethanol, Tetrahydrofuran, Methyl-tert.-butylether oder N-Methylpyrrolidon durchführen. Im Lösungsmittel können dabei das Nitril der allgemeinen Formel (II) und/oder das sekundäre Amin der allgemeinen Formel (III) und/oder bei der Umsetzung entstehender Ammoniak gelöst sein. Vorzugsweise wird lösungsmittelfrei gearbeitet.

Die im erfindungsgemäßen Verfahren erhaltenen Amine der allgemeinen Formel (I) lassen sich in an sich bekannter Weise, beispielsweise destillativ vom Reaktionsgemisch abtrennen und reinigen.

Im erfindungsgemäßen Verfahren werden Nitrile der allgemeinen Formel (II) umgesetzt.

(R¹R²N-CH₂-)ₙ₋ₘX(-CN)ₘ (II)

- X: ist dabei ein gegebenenfalls durch C₁₋₂₀-Alkyl, C₃₋₈-Cycloalkyl, C₄-₂₀-Alkylcycloalkyl, C₄₋₂₀-Cycloalkylalkyl, C₂₋₂₀-Alkoxyalkyl, Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy, C₂₋₈-Alkoxycarbonyl substituiertes C₁₋₂₀-Alkyl, C₂₋₂₀-Alkenyl oder C₃₋₈-Cycloalkyl mit n freien Valenzen und
- n: ist eine ganze von 1 bis 4.

X ist dabei vorzugsweise C₁₋₁₂-, besonders bevorzugt C₁₋₈-, insbesondere C₁₋₆-, speziell C₁₋₄-Alkyl, das verzweigt oder unverzweigt sein kann und vorzugsweise unverzweigt ist. Beispiele sind unverzweigte Reste aus 1, 2, 3, 4, 5, 6, 7, 8, 9, 10, 11, 12, 13, 14, 15, 16, 17, 18, 19, 20 Methyleneinheiten, C(C)-C-C, C-C(C)-C, C-C(C)₂-C als Struktureinheiten. Bevorzugt sind als Struktureinheiten C, C-C, C-C-C, C-C-C-C, C-C-C-C-C-C, C-C(C)-C-C, C-C(C)-C-C, C-C-C(CN)-C-C-C, besonders bevorzugt C, C-C, C-C-C, C-C-C-C.

X kann wie vorstehend angegeben substituiert sein. Dabei kann die Anzahl der Substituenten bis zur Anzahl der substituierbaren Wasserstoffatome in X betragen. Abhängig von der Art des Restes können 1 bis 5, vorzugsweise 1 bis 3, insbesondere 0, 1 oder 2 Substituenten vorliegen. Als Substituenten kommen in Betracht:
- C₁₋₂₀-Alkoxy, bevorzugt C₁₋₈-Alkoxy wie Methoxy, Ethoxy, n-Propoxy, iso-Propoxy, n-Butoxy, iso-Butoxy, sec.-Butoxy, tert.-Butoxy, n-Pentoxy, iso-Pentoxy, sec.-Pentoxy, neo-Pentoxy, 1,2-Dimethylpropoxy, n-Hexoxy, iso-Hexoxy, sec.-Hexoxy, n-Heptoxy, iso-Heptoxy, n-Octoxy, iso-Octoxy, besonders bevorzugt C₁₋₄-Alkoxy wie Methoxy, Ethoxy, n-Propoxy, iso-Propoxy, n-Butoxy, iso-Butoxy, sec.-Butoxy und tert.-Butoxy,
- Hydroxy,
- C₁₋₂₀-Hydroxyalkyl, bevorzugt C₁₋₈-Hydroxyalkyl, besonders bevorzugt C₁₋₄-Hydroxyalkyl wie Hydroxymethyl, 1-Hydroxyethyl, 2-Hydroxyethyl, 2-Hydroxy-2-propyl und 3-Hydroxy-n-propyl,
- Amino,
- C₁₋₂₀-Alkylamino, bevorzugt C₁₋₈-Alkylamino besonders bevorzugt C₁₋₄-Alkylamino wie Methylamino, oder entsprechende Aminoalkyl, 1-Aminoethyl, 2-Aminoethyl, 2-Amino-n-propyl und 3-Amino-n-propyl,
- C₂₋₂₀-Dialkylamino, bevorzugt C₂₋₁₂-Dialkylamino, besonders C₂₋₈-Dialkylamino wie N,N-Dimethylamino, N,N-Diethylamino, N,N-Dipropylamino, N,N-Di-(1-methylethyl)amino, N,N-Dibutylamino, N,N-Di-(1-methylpropyl)-amino, N,N-Di-(2-methylpropyl)amino, N,N-Di-(1,1-dimethylethyl)-amino, N-Ethyl-N-methylamino, N-Methyl-N-propylamino, N-Methyl-N-(1-methylethyl)-amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)-amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, n-(1-Methylethyl)-N-(1-methylpropyl)amino, N-(1 Methylethyl)-N-(1-methpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, n-(1,1-Dimethylethyl)-N-(1-methylpropyl)amino und N-(1,1-Diemthylethyl)-N-(2-methylpropyl)amino,
- C₃₋₁₂-Azacycloalkyl, bevorzugt C₃₋₈-Azacycloalkylamino, besonders bevorzugt C₅₋₈-Azacycloalkyl wie Pyrrolidin, Piperidin, Azepan, Piperazin, N-Alkylpiperazin und Morpholin,
- C₃₋₈-Cycloalkylamino wie Cyclopropylamino, Cyclobutylamino, Cyclopentylamino, Cyclohexylamino, Cycloheptylamino und Cyclooctylamino, bevorzugt Cyclopentylamino, Cyclohexylamino und Cyclooctylamino, besonders bevorzugt Cyclopentylamino und Cyclohexylamino,
- C₃₋₈-Dicycloalkylamino,
- Arylamino wie Phenylamino, 1-Naphthylamino und 2-Naphthylamino, bevorzugt Phenylamino,
- Aryl-C₁₋₈-alkylamino, bevorzugt Phenyl-C₁₋₈-alkylamino, besonders bevorzugt Phenyl-C₁₋₄-alkylamino wie Phenylmethylamino und Phenylethylamino,
- Halogen, bevorzugt Fluor, Chlor und Brom, besonders bevorzugt Fluor und Chlor,
- Mercapto,
- C₂₋₂₀-Oxacycloalkyl, bevorzugt C₂₋₈-Oxacycloalkyl, besonders bevorzugt C₂₋₈-Oxacycloalkyl, wie 2-Tetrahydrofuranyl, 3-Tetrahydrofuranyl, 2-Furanyl und 3-Furanyl,
- C₃₋₈-Cycloalkoxy wie Cyclopropoxy, Cyclobutoxy, Cyclopentoxy, Cyclohexoxy, Cycloheptoxy und Cyclooctoxy, bevorzugt Cyclopentoxy, Cyclohexoxy, besonders bevorzugt Cyclopentoxy und Cyclohexoxy,
- Aryloxy wie Phenoxy, 1-Naphthoxy und 2-Naphthoxy, bevorzugt Phenoxy.

Vorzugsweise liegen 0, 1 oder 2 Substituenten vor, die OH oder C₂₋₁₂-, vorzugsweise C₂₋₆, insbesondere C₂₋₄-Dialkylamino sind. Insbesondere sind die Substituenten Dimethylamino oder OH.

R¹ und R² bedeuten unabhängig C₁₋₂₀₀-Alkyl, C₃₋₈-Cycloalkyl, C₄₋₂₀-Alkylcycloakyl, C₄₋₂₀-Cycloalkylalkyl, C₂₋₂₀-Alkoxyalkyl, Aryl, C₇₋₂₀-Alkylaryl, C₇₋₂₀-Aralkyl, C₂₋₈-Hydroxyalkyl, C₂₋₈-Mercaptoalkyl, C₈₋₂₀-Aryloxyalkyl oder gemeinsam eine gegebenenfalls ein- bis dreifach durch C₁₋₄-Alkyl substituierte gesättigte oder ungesättigte gegebenenfalls durch Sauerstoff unterbrochene C₂₋₆-Alkylenkette. Dabei sind folgende Reste bevorzugt:
- C₁₋₂₀₀-Alkyl, vorzugsweise C₁₋₂₀-Alkyl, bevorzugt C₁₋₁₂-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, n-Nonyl, iso-Nonyl, n-Decyl, iso-Decyl, n-Undecyl, n-Dodecyl und iso-Dodecyl, besonders bevorzugt C₁₋₄-Alkyl wie Methyl, Ethyl, n-Propyl, iso-Propyl-, n-Butyl-, iso-Butyl, sec.-Butyl-, tert.-Butyl sowie bevorzugt C₄₀₋₂₀₀-Alkyl wie Polybutyl, Polyisobutyl, Polypropyl, Polyisopropyl und Polyethyl, besonders bevorzugt Polybutyl und Polyisobutyl, Propyl, iso-Propyl, n-Butyl, iso-Butyl, sec.-Butyl und tert.-Butyl,
- C₃₋₈-Cycloalkyl, wie Cyclopropyl, Cyclobutyl, Cyclopentyl, Cyclohexyl, Cycloheptyl und Cyclooctyl, bevorzugt Cyclopentyl, Cyclohexyl und Cyclooctyl, besonders bevorzugt Cyclopentyl und Cyclohexyl,
- C₄₋₂₀-Alkylcycloalkyl, bevorzugt C₄₋₁₂-Alkylcycloalkyl,
- C₄₋₂₀-Cycloalkylalkyl, bevorzugt C₄₋₁₂-Cycloalkylalkyl,
- C₂₋₂₀-Alkoxyalkyl, bevorzugt C₂₋₈-Alkoxyalkyl wie Methoxymethyl, 2-Methoxyethyl, 2-Ethoxyethyl, 3-Methoxypropyl und 3-Ethoxypropyl,
- Aryl wie Phenyl, 1-Naphthyl und 2-Naphthyl, bevorzugt Phenyl,
- C₇₋₂₀-Alkylaryl wie C₇₋₂₀-Alkylphenyl, bevorzugt C₇₋₁₂-Alkylphenyl,
- C₇₋₂₀-Aralkyl, bevorzugt C₇₋₁₂-Phenylalkyl wie Benzyl, 1-Phenethyl, 2-Phenethyl, 1-Phenylpropyl, 2-Phenylpropyl, 3-Phenylbutyl, 1-Phenylbutyl, 1-Phenylbutyl, 3-Phenylbutyl und 4-Phenylbutyl, besonders bevorzugt Benzyl, 1-Phenethyl und 2-Phenethyl,
- C₂₋₈-Hydroxyalkyl, bevorzugt C₂₋₄-Hydroxyalkyl wie 1-Hydroxyethyl, 2-Hydroxyethyl, 2-Hydroxy-n-propyl und 3-Hydroxy-n-propyl,
- C₂₋₈-Mercaptoalkyl, bevorzugt C₂₋₄-Mercaptoalkyl wie 1-Mercaptoethyl, 2-Mercaptoethyl, 2-Mercapto-n-propyl und 3-Mercapto-n-propyl,
- C₈₋₂₀-Phenoxyalkyl, bevorzugt C₈₋₁₂-Phenoxyalkyl wie 2-Phenoxyethyl, 2-Phenoxypropyl, 3-Phenoxypropyl, 2-Phenoxybutyl, 3-Phenoxybutyl und 4-Phenoxybutyl, besonders bevorzugt 2-Phenoxyethyl,
- gemeinsam eine gegebenenfalls ein- bis dreifach durch C₁₋₄-Alkyl substituierte gesättigte oder ungesättigte gegebenenfalls durch Sauerstoff unterbrochene C₂₋₆-Alkylenkette wie -CH₂-O-CH₂-CH₂-, -CH₂-CH₂-O-CH₂-CH₂-, -CH₂-CH₂-O-CH₂-CH₂-, -CH₂-CH₂-CH₂-O-CH₂-CH₂-CH₂-, und -CH₂-CH(CH₃)-CH(CH₃)-CH₂-.

Die am meisten bevorzugten Reste R¹ und R² sind C₁₋₆-Alkylreste, insbesondere Methyl- oder Ethylreste.

n ist eine ganze Zahl von 1 bis 4, vorzugsweise 1 bis 3, insbesondere 1 oder 2.

m ist eine ganze Zahl von 1 bis n, vorzugsweise gleich n.

Die Umsetzung erfolgt mit sekundären Aminen der allgemeinen Formel (III)

HNR¹R² (III)

in der R¹ und R² die oben angegebenen Bedeutungen haben.

Bevorzugte Nitrile der allgemeinen Formel (II) sind Acetonitril, Propionitril, Isopropionitril, Butyronitril, Valeronitril, Pentensäurenitril, Retensäurenitril, 3-Hydroxypropionitril, 3-Methoxypropionitril, 3-Ethoxypropionitril, 3-Propoxypropionitril, 3-Isopropoxypropionitril, 3-Cyclohexoxypropionitril, 2-Methyl-3-hydroxypropionitril, 3-Methoxy-2-methylpropionitril, 3-Ethoxy-2-methylpropionitril, 2-Methyl-3-propoxypropionitril, 3-Isopropoxy-2-methylpropionitril, 3-Cyclohexoxy-2-methylpropionitril, 3-Methyl-3-Hydroxypropionitril, 3-Methoxy-3-methylpropionitril, 3-Ethoxy-3-methylpropionitril, 3-Methyl-3-propoxypropionitril, 3-Isopropoxy-3-methylpropionitril, 3-Cyclohexoxy-3-methyl-propionitril, 3-Aminopropionitril, 3-Methylaminopropionitril, 3-Dimethylaminopropionitril, 3-Ethylaminopropionitril, 3-Diethylaminopropionitril, 3-Propylaminopropionitril, 3-Dipropylaminopropionitril, 3-Isopropylaminopropionitril, 3-Diisopropylaminopropionitril, 3-Cyclohexylaminopropionitril, 3-Dicyclohexylaminopropionitril, N-(Cyanoethyl)-N-methylanilin. Besonders bevorzugt sind 3-Hydroxypropionitril, 3-Methoxypropionitril, 3-Dimethylaminopropionitril, 3-Diethylaminopropionitril, 3-Cyclohexylaminopropionitril und 3-Methylaminopropionitril, insbesondere Biscyanethylether, Biscyanethylamin, N-Methyl-biscyanethylamin, N-Ethyl-biscyanethylamin, N-n-Propyl-biscyanethylamin, N-n-Propyl-biscyanethylamin, Polyisobutylennitril, N-Polyisobutylenaminopropionitril, Triscyanethylamin, 5-Aminovaleriansäurenitril, 5-Methylaminovaleriansäurenitril, 5-Dimethylaminovaleriansäurenitril, 6-Aminocapronsäurenitril, 6-Methylaminocapronsäurenitril, 6-Dimethylaminocapronsäurenitril, 5-Amino-4-methylvaleriansäurenitril, 5-Methyl-amino-4-methylvaleriansäurenitril, 5-Dimethylamino-4-methylvaleriansäurenitril, 5-Ethylamino-4-methylvaleriansäurenitril, 5-Diethylamino-4-methylvaleriansäurenitril, 5-Amino-2-methylvaleriansäurenitril, 5-Methylamino-2-methylvaleriansäurenitril, 5-Dimethylamino-2-valeriansäurenitril, 5-Ethylamino-2-methylvaleriansäurenitril, 5-Diethylamino-2-methylvaleriansäurenitril, 4-Cyanokorksäuredinitril.

Bevorzugt sind Korksäuredinitril, Adipodinitril, Methylglutarsäuredinitril, Methylenglutarsäuredinitril, Glutarsäuredinitril, Bernsteinsäuredinitril, Malonsäuredinitril, 1,2,6-Tricyanohexan. Insbesondere bevorzugt sind Adipodinitril, 3-Dimethylaminopropionitril und 3-Hydroxypropionitril.

Bevorzugte sekundäre Amine der allgemeinen Formel (III) sind:

Dimethylamin, Diethylamin, Dipropylamin, Diisopropylamin, Di-n-butylamin, Diisobutylamin, Di-sec.-butylamin, Di-2-ethylhexylamin, Di-tridecylamin, Dicyclohexylamin, Ethylmethylamin, Methylcyclohexylamin, Ethylcyclohexylamin, Piperazin, N-Methylpiperazin, N-Ethylpiperazin, Diphenylamin, N-Methylanilin, N-Ethylanilin, Diethanolamin, Di-2-methoxyethylamin, Di-2-ethoxyethylamin, Methylethanolamin, Ethylethanolamin, Isopropylethanolamin, Hydroxyethylanilin. Besonders bevorzugt sind Dimethylamin, Diethylamin und Piperazin.

Die tertiären Amine I, vorzugsweise Tetramethylhexamethylendiamin, Tetramethylpropylendiamin, 3-Dimethylaminopropanol sind Härter für Epoxyharze, Katalysatoren für Polyurethane, Zwischenprodukte zur Herstellung quartärer Ammoniumverbindungen, Weichmacher, Korrosionsinhibitoren, Textilhilfsmittel, Farbstoffe und Emulgatoren. Mehrfach funktionalisierte tertiäre Amine dienen außerdem zur Herstellung von Kunstharzen, Ionenaustauschern, Pharmazeutika, Pflanzenschutz- und Schädlingsbekämpfungsmitteln.

Die Erfindung wird durch die nachstehenden Beispiele näher erläutert.

### BEISPIELE

### BEISPIEL 1

In einem Rohrreaktor (12 mm Ringspalt; 2.000 mm Länge), der mit 800 ml Katalysator (0,9% Pd, 0,1% Pt auf Zirkondioxid; 4 mm Stränge) gefüllt ist, werden stündlich von unten nach oben 160 ml/h Adipodinitril (ADN)und 215 g/h flüssiges Dimethylamin (DMA) (2,2 mol DMA/mol ADN) gepumpt. Gleichzeitig leitet man 300 Nl/h Wasserstoff bei 200 bar ein. Die Reaktionstemperatur beträgt 120°C. Nach Entspannen auf Normaldruck wird das überschüssige Dimethylamin und das gebildete Ammoniak abdestilliert. Der verbleibende Hydrieraustrag von 225 g/h enthält nach gaschromatographischer Analyse 92% Tetramethylhexamethylendiamin. Die destillativ bestimmte Ausbeute beträgt 90%. Der Adiponitril-Umsatz ist auch nach 800 h Laufzeit noch quantitativ.

### BEISPIEL 2

In den Rohreaktor aus Beispiel 1 werden 500 ml des gleichen Katalysators eingefüllt und in Sumpffahrweise 65 ml/h Ethylencyanhydrin sowie 70 g/h DMA (Molverhältnis 1:1.6) darübergeleitet. Die Reaktortemperatur betrug 120°C und der Wasserstoffdruck 200 bar. Nach Entspannung auf Normaldruck sowie Entfernung von Ammoniak/DMA wurden 89 GC-Fl-% Dimethylaminopropanol erhalten. Die Anfangsbelastung von 0,13 l/lh wurde dann im Laufe von 1000 h schrittweise auf 0,5 l/lh gesteigert, wobei die Temperatur auf 160 °C erhöhr wurde, so daß auch gegen Ende des Versuches noch 90% Dimethylaminopropanol anfielen. Es tritt keine Desaktivierung auf, der Katalysator wird mechanisch gut erhalten ausgebaut.

## Patentansprüche

1. Verfahren zur Herstellung von Aminen der allgemeinen Formel (I)
X(-CH₂-NR¹R²)ₙ (I)
in der
R¹, R² unabhängig C₁₋₂₀₀-Alkyl, C₃₋₈-Cycloalkyl, C₄₋₂₀-Alkylcycloakyl, C₄₋₂₀-Cycloalkylalkyl, C₂₋₂₀-Alkoxyalkyl, Aryl, C₇-₂₀-Alkylaryl, C₇₋₂₀-Aralkyl, C₂₋₈-Hydroxyalkyl, C₂₋₈-Mercaptoalkyl, C₈₋₂₀-Aryloxyalkyl oder gemeinsam eine gegebenenfalls ein- bis dreifach durch C₁₋₄-Alkyl substituierte gesättigte oder ungesättigte gegebenenfalls durch Sauerstoff unterbrochene C₂₋₆-Alkylenkette bedeuten
X ein gegebenenfalls durch C₁₋₂₀-Alkyl, C₃₋₈-Cycloalkyl, C₄₋₂₀-Alkylcycloalkyl, C₄₋₂₀-Cycloalkylalkyl, C₂₋₂₀-Alkoxyalkyl, Aryl, C₇₋₂₀-Alkylaryl, C₇₋₂₀-Aralkyl, C₁₋₂₀-Alkoxy, Hydroxy, C₁₋₂₀-Hydroxyalkyl, Amino, C₁₋₂₀-Alkylamino, C₂₋₂₀-Dialkylamino, C₂₋₁₂-Alkenylamino, C₃₋₈-Cycloalkylamino, Arylamino, Diarylamino, Aryl-C₁₋₈-alkylamino, Halogen, Mercapto, C₂₋₂₀-Alkenyloxy, C₃₋₈-Cycloalkoxy, Aryloxy, C₂₋₈-Alkoxycarbonyl substituiertes C₁₋₂₀-Alkyl, C₂₋₂₀-Alkenyl oder C₃₋₈-Cycloalkyl mit n freien Valenzen ist und
n eine ganze Zahl von 1 bis 4 ist,
durch Umsetzung von Nitrilen der allgemeinen Formel (II)
(R¹R²N-CH₂-)ₙ₋ₘX(-CN)ₘ (II)
in der R¹, R² und X die oben angegebenen Bedeutungen haben und m eine ganze Zahl von 1 bis n ist,
mit sekundären Aminen der allgemeinen Formel (III)
HNR¹R² (III)
in der R¹ und R² die oben angegebenen Bedeutungen haben,
und Wasserstoff bei Temperaturen von 50 bis 250°C und Drücken von 5 bis 350 bar in Gegenwart eines Pd enthaltenden Katalysators, wobei der Katalysator, bezogen auf das Gesamtgewicht des Katalysators 0,5 bis 1 Gew.-% Pd und 0,05 bis 0,2 Gew.-% platin oder 1 bis 5 Gew.-%. Lanthan, auf einem Träger enthält.

2. Verfahren nach Anspruch 1, wobei der Träger ausgewählt ist aus Aktivkohle, Siliciumcarbid und Metalloxiden.

3. Verfahren nach Anspruch 2, wobei der Träger ausgewählt ist aus ZrO₂, Al₂O₂, SiO₂, TiO₂ oder Gemischen davon.

4. Verfahren nach einem der Ansprüche 1 bis 3, wobei n den Wert 1 oder 2 hat.

5. Verfahren nach einem der Ansprüche 1 bis 4, wobei m den gleichen Wert wie n hat.

6. Verfahren nach einem der Ansprüche 1 bis 5, wobei X lineares C₁₋₆-Alkyl mit bis zu 2 Substituenten an X ist.

7. Verfahren nach Anspruch 7, wobei die Substituenten C₂₋₁₂-Dialkylamino oder OH sind.

8. Verfahren nach einem der Ansprüche 1 bis 7, wobei R¹ und R² un-abhängig C₁₋₆-Alkyl sind.

9. Verwendung eines Katalysators, wie er in einem der Ansprüche 1 bis 34 definiert ist, zur Umsetzung von sekundären Aminen mit Nitrilen und Wasserstoff zu tertiären Aminen.

## Claims

1. A process for preparing amines of the formula (I)
X(-CH₂-NR¹R²)ₙ (I)
where
R¹ and R² are, independently, C₁₋₂₀₀-alkyl, C₃₋₈ -cycloalkyl, C₄₋₂₀-alkylcycloalkyl, C₄₋₂₀ -cycloalkylalkyl, C₂₋₂₀-alkoxyalkyl, aryl, C₇₋₂₀-alkylaryl, C₇₋₂₀-aralkyl, C₂₋₈ -hydroxyalkyl, C₂₋₈-mercaptoalkyl, C₈₋₂₀ -aryloxyalkyl or together are a saturated or unsaturated C₂₋₆-alkylene chain which is unsubstituted or substituted one to three times by C₁₋₄-alkyl and may be in-terrupted by oxygen,
X is unsubstituted or C₁₋₂₀-alkyl-, C₃₋₈ -cycloalkyl-, C₄₋₂₀-alkylcycloalkyl-, C₄₋₂₀ -cycloalkylalkyl-, C₂₋₂₀-alkoxyalkyl-, aryl-, C₇₋₂₀-alkylaryl-, C₇₋₂₀-aralkyl-, C₁₋₂₀-alkoxy-, hydroxy-, C₁₋₂₀-hydroxy-alkyl-, amino-, C₁₋₂₀-alkylamino-, C₂₋₂₀ -dialkylamino-, C₂₋₁₂-alkenylamino-, C₃₋₈ -cycloalkylamino-, arylamino-, diaryl-amino-, aryl-C₁₋₈-alkylamino-, halo-, mercapto-, C₂₋₂₀-alkenyloxy-, C₃₋₈-cycloalkoxy-, aryloxy-, C₂₋₈-alkoxycarbonylsubstituted C₁₋₂₀-alkyl, C₂₋₂₀-alkenyl or C₃₋₈-cycloalkyl with n free valencies, and
n is an integer from 1 to 4,
by reacting nitriles of the formula (II)
(R¹R²N-CH₂-)ₙ₋ₘX(-CN)ₘ (II)
where R¹, R² and X have the abovementioned meanings, and m is an integer from 1 to n,
with secondary amines of the formula (III)
HNR¹R² (III)
where R¹ and R² have the abovementioned meanings,
and hydrogen at from 50 to 250°C under pressures of from 5 to 350 bar in the presence of a Pd-containing catalyst, where the catalyst comprises, based on the total weight of the catalyst, from 0.5 to 1% by weight Pd and from 0.05 to 0.2% by weight of platinum or from 1 to 5% by weight of lanthanum on a carrier.

2. A process as claimed in claim 1, wherein the carrier is selected from active carbon, silicon carbide and metal oxides.

3. A process as claimed in claim 2, wherein the carrier is selected from ZrO₂, Al₂O₃, SiO₂, TiO₂ or mixtures thereof.

4. A process as claimed in any of claims 1 to 3, where n is 1 or 2.

5. A process as claimed in any of claims 1 to 4, where m has the same value as n.

6. A process as claimed in any of claims 1 to 5, where X is linear C₁₋₆-alkyl with up to 2 substituents on X.

7. A process as claimed in claim 6, where the substituents are C₂₋₁₂-dialkylamino or OH.

8. A process as claimed in any of claims 1 to 7, where R¹ and R² are, independently, C₁₋₆-alkyl.

9. The use of a catalyst as defined in any of claims 1 to 3 for reacting secondary amines with nitriles and hydrogen to give tertiary amines.

## Revendications

1. Procédé pour la préparation d'amines de formule générale (I)
X(-CH₂-NR¹R²)ₙ (I)
dans laquelle
R¹, R² représentent, indépendamment l'un de l'autre, un groupe alkyle en C₁ à C₂₀₀, cycloalkyle en C₃ à C₈, alkylcycloacyle en C₄ à C_{20,} cycloalkylalkyle en C₄ à C₂₀, alcoxyalkyle en C₂ à C₂₀, aryle, alkylaryle en C₇ à C₂₀, aralkyle en C₇ à C₂₀, hydroxyalkyle en C₂ à C₈, mercaptoalkyle en C₂ à C₈, aryloxyalkyle en C₈ à C₂₀, ou bien ils représentent conjointement une chaîne alkylène en C2 à C₆, saturée ou insaturée, qui est éventuellement substituée, une à trois fois, par un groupe alkyle en Ci à C₄, et qui est éventuellement interrompue par un atome d'oxygène,
X représente un groupe alkyle en C₁ à C₂₀, alcényle en C₂ à C₂₀ ou cycloalkyle en C₃ à C₈ qui présente n valences libres et qui est éventuellement substitué par un groupe alkyle en C₁ à C₂₀, cycloalkyle en C₃ à C₈, alkylcycloalkyle en C₄ à C₂₀, cycloalkylalkyle en C₄ à C₂₀, alcoxyalkyle en C₂ à C₂₀, aryle, alkylaryle en C₇ à C₂₀, aralkyle en C₇ à C₂₀, alcoxy en C₁ à C₂₀, hydroxy, hydroxyalkyle en C₁ à C₂₀, amino, alkylamino en C₁ à C₂₀, dialkylamino en C₂ à C₂₀, alcénylamino en C₂ à C₁₂, cycloalkylamino en C₃ à C₈, arylamino, diarylamino, aryl-(alkyle en C₁ à C₈)amino, un atome d'halogène, un groupe mercapto, alcényloxy en C₂ à C₂₀, cycloalcoxy en C₃ à C₈, aryloxy ou alcoxycarbonyle en C₂ à C₈, et
n représente un nombre entier allant de 1 à 4,
au moyen de la réaction des nitriles de formule générale (II)
(R¹R²N-CH₂-)ₙ₋ₘX(-CN)ₘ (II)
dans laquelle R¹, R² et X prennent la signification susmentionnée, et dans laquelle m représente un nombre entier allant de 1 à n,
avec des amines secondaires de formule générale (III)
HNR¹R² (III)
dans laquelle R¹ et R² prennent la signification susmentionnée,
et avec l'hydrogène, à une température allant de 50°C à 250°C et sous une pression allant de 5 bars à 350 bars, en présence d'un catalyseur contenant Pd, le catalyseur contenant, sur un support, 0,5% à 1% en poids de Pd et 0,05% à 0,2% en poids de platine ou 1% à 5% en poids de lanthane, par rapport au poids total du catalyseur.

2. Procédé selon la revendication 1, dans lequel le support est choisi dans le groupe formé par le charbon actif, le carbure de silicium et les oxydes métalliques.

3. Procédé selon la revendication 2, dans lequel le support est choisi dans le groupe formé par ZrO₂, Al₂O₂, SiO₂, TiO₂ et leurs mélanges.

4. Procédé selon l'une quelconque des revendications 1 à 3, dans lequel n vaut 1 ou 2.

5. Procédé selon l'une quelconque des revendications 1 à 4, dans lequel m est égal à n.

6. Procédé selon l'une quelconque des revendications 1 à 5, dans lequel X représente un groupe alkyle en C₁ à C₆ linéaire possédant jusqu'à 2 substituants sur X.

7. Procédé selon la revendication 6, dans lequel les substituants sont un groupe dialkylamino en C₂ à C₁₂ ou un groupe OH.

8. Procédé selon l'une quelconque des revendications 1 à 7, dans lequel R¹ et R² représentent, indépendamment l'un de l'autre, un groupe alkyle en C₁ à C₆.

9. Mise en oeuvre d'un catalyseur tel qu'il est défini selon l'une quelconque des revendications 1 à 3, pour réaliser la réaction des amines secondaires, avec des nitriles et de l'hydrogène, en amines tertiaires.
